# FASCICULE DE BREVET EUROPEEN

(11) **EP 0 937 721 B1**
(45) Date de publication et mention de la délivrance du brevet: **26.09.2001**
(21) Numéro de dépôt: 99400290.5
(22) Date de dépôt: 09.02.1999
(51) Int. Cl.: C07D 333/10, C07B 63/00

(54) **Purification du thiophène**
Reinigung von Thiophen
Purification of thiophene

(30) Priorité: 18.02.1998 FR 9801972
(43) Date de publication de la demande: 25.08.1999
(73) Titulaire: Atofina, 92800 Puteaux (FR)
(72) Inventeur: Labat, Yves, 33110 Le Bouscat (FR); Luyendijk, Piet, 3233 AB Oostvoorne (NL)
(74) Mandataire: Leboulenger, Jean

(56) Documents cités:
- WO-A-97/21673
- US-A- 3 197 483
- US-A- 3 939 179

## Description

La présente invention concerne le domaine du thiophène et a plus particulièrement pour objet sa purification.

La synthèse du thiophène à partir de matières premières en C₄ telles que le furanne, le crotonaldéhyde, le butanol ou le butane, et de composés soufrés tels que le disulfure de carbone, le sulfure d'hydrogène ou le soufre peut s'accompagner de la formation de mercaptans en C₄ (butylmercaptan primaire ou secondaire) qu'il est difficile de séparer par distillation car leurs points d'ébullition sont proches de celui du thiophène. D'autre part, la présence de faibles quantités de mercaptans peut modifier les caractéristiques olfactives du thiophène commercial et est donc indésirable.

Il est connu de purifier les hydrocarbures contenant des impuretés soufrées par adsorption sur tamis, résines et adsorbants divers. Il est également connu d'oxyder par l'air ou l'oxygène les mercaptans contenus dans les hydrocarbures. Malheureusement ces techniques ne sont pas applicables au thiophène qui peut, lui-même, être oxydé en sulfoxydes et/ou sulfones.

On peut également utiliser d'autres techniques comme l'extraction par solvants, en particulier par une solution aqueuse de soude. Cependant, outre sa faible efficacité et la nécessité d'utiliser un large excès de soude non recyclable, l'application de cette méthode entraînerait la formation d'effluents aqueux pollués.

Il est bien connu (voir par exemple la publication WO-A-97/21673) de fabriquer des polysulfures par action du soufre sur des mercaptans en présence d'un catalyseur basique.

Il a maintenant été trouvé que l'oxydation des mercaptans contenus dans le thiophène par le soufre en présence d'un catalyseur basique est rapide et pratiquement complète, et permet de former des polysulfures lourds aisément séparables par distillation du thiophène.

L'invention a donc pour objet un procédé de purification d'un thiophène contaminé par des mercaptans caractérisé en ce qu'il consiste à assurer l'oxydation sélective des mercaptans en polysulfures, puis à distiller le thiophène ainsi traité.

L'oxydation des mercaptans par le soufre nécessite l'emploi d'un catalyseur basique. L'utilisation d'une solution aqueuse de soude, sans addition de soufre, peut aussi être envisagée, mais elle conduit à la formation d'effluents aqueux.

Les catalyseurs basiques permettant l'oxydation des mercaptans par le soufre sont bien connus et peuvent tous être utilisés dans la mise en oeuvre du procédé selon l'invention. Les catalyseurs basiques liquides sont utilisables, mais ils présentent l'inconvénient d'être difficilement recyclables dans une nouvelle opération.

C'est pourquoi, conformément à un aspect préférentiel de l'invention, on choisit avantageusement le catalyseur basique parmi les catalyseurs solides car, à la fin de la réaction, ils sont aisément séparables par filtration et donc réutilisables pour une autre opération de purification. En utilisant un catalyseur solide, la première étape du procédé selon l'invention peut aussi être réalisée en continu par circulation forcée des réactifs (soufre et thiophène à purifier) au travers d'une colonne remplie de catalyseur solide ; il n'est alors même plus nécessaire de filtrer ce dernier en fin de réaction.

Comme exemples non limitatifs de catalyseurs basiques utilisables pour l'oxydation des mercaptans en polysulfures par réaction avec le soufre, on peut citer :
- les alumines, titanates, silices ou mélanges de ces composés, éventuellement modifiés par des alcalis ou des bases alcalino-terreuses ;
- des zéolithes ou hydrocalcites ;
- des oxydes ou sels métalliques comme Na₂O, K₂O, NaHCO₃, ZnO, MgO, ZrO₂ et CaCO₃, ces composés pouvant être utilisés tels quels ou, pour certains d'entre eux, fixés sur un support (par exemple, une alumine) ;
- des mercaptides ou alcoolates tels que RSNa, RS(CH₂CH₂O)ₙNa et RO(CH₂CH₂O)ₙNa, R représentant un radical hydrocarbyle en C₁ à C₁₂ et n un nombre entier allant de 1 à 10 ;
- des amines, hydroxydes d'ammonium, alcanolamines ou hydroxydes métalliques tels que LiOH, NaOH et KOH.

Conformément à un autre aspect préférentiel de l'invention, on utilise comme catalyseur solide une résine basique échangeuse d'anions telle que, par exemple, une résine à base d'un copolymère styrène-divinylbenzène fonctionnalisé par des groupements amino primaire, secondaire ou tertiaire ou par des groupements guanidine ou amidine. Ces résines basiques, bien connues, sont disponibles dans le commerce (par exemple, celles à fonction amine tertiaire commercialisées par Rohm et Haas sous la dénomination Amberlyst® ) ou sont décrites dans la littérature (brevets FR 2 742 144, FR 2 742 145 et FR 2 742 157).

Le traitement d'oxydation selon l'invention est réalisé par dissolution dans le thiophène à traiter d'une quantité de soufre qui peut aller de 1 à 10 atomes-gramme de soufre par mole de mercaptan présent, de préférence de 2 à 4.

La réaction d'oxydation peut être effectuée à une température allant de la température ambiante jusqu'à 120°C, mais on travaille de préférence à une température comprise entre 60 et 90°C qui permet de conserver le thiophène à l'état liquide et, en même temps, d'évacuer le sulfure d'hydrogène produit par la réaction d'oxydation des mercaptans par le soufre. Au delà de 90°C, il faut opérer sous pression, celle-ci ne devant cependant pas être trop élevée de façon à permettre l'élimination du sulfure d'hydrogène.

La quantité de catalyseur basique à utiliser peut varier dans de larges limites. Par rapport au poids de thiophène à purifier, elle est généralement comprise entre 0,1 et 10 % pour un catalyseur liquide et entre 0,5 et 15 % pour un catalyseur solide.

On peut opérer en discontinu (batch), le temps de réaction dépendant de divers paramètres tels que l'agitation, la quantité de catalyseur employée et sa nature chimique. En batch, une à trois heures de réaction suffisent généralement pour convertir 95 % des mercaptans présents dans le thiophène ; une meilleure conversion peut être obtenue en prolongeant le temps de réaction.

On peut aussi opérer en continu en faisant circuler dans une colonne garnie d'un lit fixe de catalyseur solide (par exemple, une résine basique) un courant du thiophène à purifier et dans lequel est préalablement dissous le soufre nécessaire à la réaction d'oxydation.

La distillation finale pour séparer le thiophène des polysulfures formés peut être effectuée de façon conventionnelle.

L'exemple suivant illustre l'invention sans la limiter.

### EXEMPLE 1

Dans un réacteur agité de 1 litre et thermostaté, on a introduit 400 g de thiophène pollué par 0,4 % de mercaptans butyliques (mélange de mercaptans primaire et secondaire), 40 g de résine Amberlyst® A21 préalablement séchée et 1,2 g de soufre, ce qui correspond à un rapport molaire S/mercaptans d'environ 2.

Après une heure d'agitation à 80°C, on avait converti 90 % des mercaptans et, après 2 heures, 95 %.

Le thiophène ainsi traité ne contenait plus que 130 ppm de mercaptans après 4 heures d'agitation. Le mélange réactionnel était refroidi et filtré pour séparer la résine qui a été utilisée pour de nouvelles opérations avec une efficacité comparable.

Après distillation du filtrat, on a obtenu un thiophène dont la teneur en mercaptans, dosés par potentiométrie, est inférieure à 100 ppm et qui a perdu toute odeur spécifique des mercaptans.

### EXEMPLE 2

Dans un réacteur de 6900 litres, on a chargé 5000 kg de thiophène pollué par 0,52 % de mercaptans butyliques et 80 kg de soufre apporté sous forme liquide. Ce mélange, chauffé à environ 85°C (température de reflux), a été mis en circulation sur un lit fixe constitué de 400 litres de résine Amberlyst® A21 préalablement lavée à l'acétone puis au thiophène.

Après environ 2,5 heures, la circulation a été coupée et on a distillé du réacteur environ 4000 kg de thiophène. Le thiophène ainsi purifié présentait une teneur en mercaptans (dosés par potentiométrie) de 300 ppm.

Aux résidus lourds de distillation restant dans le réacteur et contenant encore du soufre en excès, on a rajouté une nouvelle charge de 4000 kg de thiophène pollué, et la procédure (circulation, puis distillation) a été répétée.

La quantité initiale de soufre a été suffisante pour traiter 6 charges d'affilée (soit au total 25000 kg de thiophène) sans rajout de soufre.

Après la sixième opération et par une distillation plus poussée, on a encore récupéré 700 kg de thiophène que l'on recycle dans une autre opération de purification ; les résidus lourds de distillation contenant des polysulfures à haut point d'ébullition, du soufre et un peu de thiophène ont été éliminés.

Après 6 campagnes de 6 charges (au total 36 charges) aucune désactivation de la résine n'a été observée.

## Revendications

1. Procédé de purification d'un thiophène contaminé par des mercaptans, **caractérisé en ce qu'**il consiste à assurer l'oxydation sélective des mercaptans en polysulfures, puis à distiller le thiophène ainsi traité.

2. Procédé selon la revendication 1 **caractérisé en ce que** la transformation des mercaptans en polysulfures est obtenue par oxydation des mercaptans par le soufre en présence d'un catalyseur basique.

3. Procédé selon la revendication 2 dans lequel on utilise un catalyseur basique solide.

4. Procédé selon la revendication 3 dans lequel le catalyseur basique solide est une résine à fonctions amine, guanidine ou amidine.

5. Procédé selon l'une des revendications 1 à 4, dans lequel l'oxydation est effectuée en utilisant 1 à 10 atomes-gramme de soufre par mole de mercaptans.

6. Procédé selon la revendication 5 dans lequel on utilise 2 à 4 atomes-gramme de soufre par mole de mercaptans.

7. Procédé selon l'une des revendication 1 à 6 dans lequel l'oxydation est effectuée à une température allant de la température ambiante jusqu'à 120°C.

8. Procédé selon la revendication 7 dans lequel la température est comprise entre 60 et 90°C.

9. Procédé selon l'une des revendications 2 et 5 à 8 dans lequel on utilise un catalyseur basique liquide en une proportion de 0,1 à 10 % par rapport au poids de thiophène à purifier.

10. Procédé selon l'une des revendication 3 à 8 dans lequel on utilise un catalyseur basique solide en une proportion de 0,5 à 15 % par rapport au poids de thiophène à purifier.

## Claims

1. Process for purifying a thiophene contaminated by mercaptans, **characterized in that** it consists in selectively oxidizing the mercaptans to form polysulphides, then in distilling the thiophene treated in this way.

2. Process according to Claim 1, **characterized in that** the conversion of the mercaptans into polysulphides is obtained by oxidizing the mercaptans with sulphur in the presence of a basic catalyst.

3. Process according to Claim 2, in which use is made of a solid basic catalyst.

4. Process according to Claim 3, in which the solid basic catalyst is a resin having amine, guanidine or amidine functional groups.

5. Process according to one of Claims 1 to 4, in which the oxidation is carried out by using from 1 to 10 gram-atoms of sulphur per mole of mercaptans.

6. Process according to Claim 5, in which from 2 to 4 gram-atoms of sulphur are used per mole of mercaptans.

7. Process according to one of Claims 1 to 6, in which the oxidation is carried out at a temperature ranging from room temperature to 120°C.

8. Process according to Claim 7, in which the temperature is comprised between 60 and 90°C.

9. Process according to one of Claims 2 and 5 to 8, in which use is made of a liquid basic catalyst in a proportion of from 0.1 to 10% relative to the weight of thiophene to be purified.

10. Process according to one of Claims 3 to 8, in which use is made of a solid basic catalyst in a proportion of from 0.5 to 15% relative to the weight of thiophene to be purified.

## Patentansprüche

1. Verfahren zur Reinigung eines mit Mercaptanen kontaminierten Thiophens, **dadurch gekennzeichnet, daß** es darin besteht, eine selektive Oxidation der Mercaptane in Polysulfide zu ermöglichen, dann das so behandelte Thiophen zu destillieren.

2. Verfahren nach Anspruch 1, **dadurch gekennzeichnet, daß** die Umwandlung der Mercaptane in Polysulfide durch Oxidation der Mercaptane durch Schwefel in Gegenwart eines basischen Katalysators erreicht wird.

3. Verfahren nach Anspruch 2, bei dem man einen festen basischen Katalysator verwendet.

4. Verfahren nach Anspruch 3, bei dem der feste basische Katalysator ein Harz mit Amin-, Guanidin- oder Amidingruppen ist.

5. Verfahren nach einem der Ansprüche 1 bis 4, bei dem die Oxidation durchgeführt wird, indem 1 bis 10 Atomgramm Schwefel pro Mol der Mercaptane verwendet wird.

6. Verfahren nach Anspruch 5, bei dem man 2 bis 4 Atomgramm Schwefel pro Mol der Mercaptane verwendet.

7. Verfahren nach einem der Ansprüche 1 bis 6, bei dem die Oxidation bei einer Temperatur durchgeführt wird, die von der Umgebungstemperatur bis zu 120 °C reicht.

8. Verfahren nach Anspruch 7, bei dem die Temperatur zwischen 60 und 90 °C liegt.

9. Verfahren nach einem der Ansprüche 2 und 5 bis 8, bei dem man einen flüssigen basischen Katalysator in einem Verhältnis von 0,1 bis 10 %, bezogen auf das Gewicht des zu reinigenden Thiophens, verwendet.

10. Verfahren nach einem der Ansprüche 3 bis 8, bei dem man einen festen basischen Katalysator in einem Verhältnis von 0,5 bis 15 %, bezogen auf das Gewicht des zu reinigenden Thiophens, verwendet.
